# EUROPEAN PATENT SPECIFICATION

(11) **EP 3 406 185 B1**
(45) Date of publication and mention of the grant of the patent: **24.02.2021**
(21) Application number: 17172207.7
(22) Date of filing: 22.05.2017
(51) Int. Cl.: A61B 5/00, A61B 5/07

(54) **IMPLANTABLE MEDICAL DEVICE AND INTRA-BONE WIRELESS COMMUNICATION SYSTEM AND METHODS**
IMPLANTIERBARE MEDIZINISCHE VORRICHTUNG UND DRAHTLOSKOMMUNIKATIONSSYSTEM UND VERFAHREN IN EINEM KNOCHEN
DISPOSITIF MÉDICAL IMPLANTABLE ET SYSTÈME ET PROCÉDÉS DE COMMUNICATION SANS FIL INTRA-OSSEUSE

(43) Date of publication of application: 28.11.2018
(73) Proprietor: Vestel Elektronik Sanayi ve Ticaret A.S., 45030 Manisa (TR); Özyegin Üniversitesi, 34794 Çekmeköy/ Istanbul (TR)
(72) Inventor: GÜLBAHAR, Burhan, 34794 Çekmeköy/Istanbul (TR); MEMISOGLU, Gorkem, 45030 Manisa (TR)
(74) Representative: Kehl, Ascherl, Liebhoff & Ettmayr Patentanwälte Partnerschaft mbB

(56) References cited:
- WO-A1-2010/068984
- US-A1- 2014 180 069
- US-B1- 6 236 889
- GULBAHAR BURHAN ET AL: "Nanoscale optical communications modulator and acousto-optic transduction with vibrating graphene and resonance energy transfer", 2017 IEEE INTERNATIONAL CONFERENCE ON COMMUNICATIONS (ICC), IEEE, 21 May 2017 (2017-05-21), pages 1-7, XP033132910, DOI: 10.1109/ICC.2017.7997036 [retrieved on 2017-07-28]

## Description

The present invention relates to an implantable medical device according to claim 1, a wireless communication system according to claim 7, and a method of wireless communication according to claim 9.

### Background of the Invention

Some known techniques of wireless communication with implanted medical devices from outside the human body are described in Ferguson J.E. & Redish A.D.: "Wireless communication with implanted medical devices using the conductive properties of the body", Expert Rev. Med. Devices, 2011 Jul; 8(4): pp. 427-433. The known techniques described in this document include different electrical modes of communication, such as capacitive, galvanic and radio frequency (RF) coupling.

Wireless communication with implanted medical devices from outside the human body using externally applied acoustic signals, instead of electrical signals, is also known, and is described, for example, at page 6, lines 25 to 29 of WO 2001/58388 A, and at page 2, lines 23 to 29 of EP 1 331 969 A. In both these cases, acousto-electric transducers implanted in the body of a subject are used to receive and convert the acoustic signals into electrical signals. However, such acousto-electric transducers have several disadvantages, including that they are generally bulky and therefore difficult to implant successfully into a subject with minimal disruption, as well as requiring their own electrical power supply.

The wireless transmission of data from biosensors implanted within a subject is also known. US 2006/224088 A, for example, describes the implantation of biosensors adjacent to the vertebrae of the spinal column of a human subject, and the transmission of data from the biosensors to outside the human subject for analysis.

However, wireless communication with medical devices implanted into bones is generally very difficult using known techniques, because neither RF nor optical signals can penetrate into bones very well, if at all. Whereas magnetic and acoustic signals can penetrate into bones, their usefulness for wireless communication with implanted medical devices is also limited in both cases by current technology, by the need for sizeable apparatus for their reception. For example, magnetic signals can be detected by a magnetic loop antenna, but as the size of such a magnetic loop antenna is decreased, so the reception strength of the signal by the antenna reduces as well. Existing acoustic devices cannot be produced at a small enough scale to make their implantation into living bones practical.

It would therefore be desirable to provide an implantable medical device, small enough to be implanted into a living bone with ease and minimal disruption, and which can nonetheless also be communicated with wirelessly from outside the body of a human or animal subject.

### Object of the Invention

It is therefore an object of the invention to provide an implantable medical device, a wireless communication system, a method of transdermal wireless communication, and a method of in vivo communication.

### Description of the Invention

The object of the invention is solved by an implantable medical device according to claim 1. The implantable medical device at least comprises an acousto-optic transducer, a photodetector, and an optical communication channel connecting an output of the acousto-optic transducer to an input of the photodetector.

This solution is beneficial because the acousto-optic transducer allows for transdermal wireless communication from outside the body of a subject using acoustic signals, the conversion of these acoustic signals into optical signals, and the onward transmission and processing of the optical signals within the body of the subject using long-established optical communication and optoelectronic techniques. The acousto-optic transducer can be made at a micro- or even nanoscale, and the photodetector can also be manufactured at a microscale. The optical communication channel connecting an output of the acousto-optic transducer to an input of the photodetector can be a very fine fibre optic, for example. Thus an implantable medical device comprising these components can be made extremely small and can be implanted into a subject, including into a living bone, with ease, and even by means of a simple injection. On the other hand, whereas the photodetector, being a passive device, requires no power supply to operate, the acousto-optic transducer can also operate on energy harvested from within the body of the subject, and therefore requires no independent power supply, either.

Moreover, the optical communication channel can also be made arbitrarily long, for example by provision of a suitable fibre optic, for communication of optical data over any distance within the body of a subject, and with minimal attenuation. Thus, the photodetector does not have to be implanted at the same location within a subject as the acousto-optic transducer, and these two elements of the implantable medical device may be separated by any distance within the body of the subject, as desired or according to needs.

Such an implantable medical device may be used in a wide variety of different applications, such as in minimally invasive surgery inside a bone during neurological operations, as well as for communication with and control of other implanted devices and prostheses.

Advantageous embodiments of the invention may be configured according to any claim and/or part of the following description.

Preferably, the acousto-optic transducer at least comprises a Förster resonance energy transfer mechanism. Förster resonance energy transfer is a process whereby energy is transferred from a first light-sensitive donor molecule to a second light-sensitive acceptor molecule. Such light-sensitive molecules are sometimes known as chromophores. In Förster resonance energy transfer (FRET), the donor molecule is firstly stimulated by incident light into an excited state. When brought sufficiently close to an acceptor molecule, the energy of the excited donor molecule is transferred to the acceptor molecule, which then emits light as a result. If the separation of the donor and acceptor molecules is modulated by an acoustic signal, then acousto-optic transduction results. The incident light may be provided by ambient light or by an artificial light source.

If the acousto-optic transducer comprises a Förster resonance energy transfer mechanism, the acousto-optic transducer preferably at least comprises a graphene resonator bearing at least one donor molecule, a substrate bearing at least one acceptor molecule, wherein the graphene resonator is responsive to sound to bring the donor molecule within range of the acceptor molecule for Förster resonance energy transfer (FRET) from the donor molecule to the acceptor molecule, an entry window arranged to permit incoming light to fall on the donor molecule, and an exit window arranged to allow light emitted by the acceptor molecule to leave the acousto-optic transducer.

Whereas the acousto-optic transducer may operate on energy harvested from within the body of a subject, if the acousto-optic transducer comprises an entry window for incoming light and other elements as just described, the implantable medical device preferably further comprises a light source to provide the incoming light and a power supply for the light source. Even so, in comparison to existing acousto-electric transducers, the wattage of the light source and consequently the energy storage capacity of the corresponding power supply can both be made tiny, so that the power may be supplied by a microcapacitor.

In various different preferred embodiments of the invention, the implantable medical device may further comprise at least one of an electronic device, a biosensor and a prosthesis, each respectively having an electrical input connected to an output of the photodetector. These solutions are beneficial because they allow for use of the implantable medical device in association with a wide variety of different electronic devices, such as can be used in surgery, in intra-bone monitoring, the repair of spinal cord and other neurological injuries, bionic prostheses, and so on.

Alternatively or additionally, the implantable medical device may comprise at least one of an optical device and an optoelectronic device, each respectively having an optical input connected to the optical communication channel. This solution is beneficial because in this manner, the implantable medical device may be integrated into an optical or optoelectronic communication system within the body of a subject.

The present invention also relates to a wireless communication system at least comprising an implantable medical device as described herein, and an electro-acoustic transducer able to transmit information to the implantable medical device by transdermal transmission of an acoustic signal.

In a further embodiment, the wireless communication system comprises a plurality of such implantable medical devices and a plurality of such electro-acoustic transducers, each able to transmit information to a respective one of the implantable medical devices by transdermal transmission of an acoustic signal, wherein the plurality of implantable medical devices are adapted for implantation into vertebrae of a spinal column, and the plurality of electro-acoustic transducers are configured for placement on a back of a human or animal subject, adjacent to their spinal column. This solution is beneficial because it can be used in the repair of spinal injuries.

The present invention further relates to a method of wireless communication, the method at least comprising transmitting an acoustic signal, receiving the acoustic signal within a human or animal subject, and converting the acoustic signal into an optical signal.

The method may be a method of transdermal wireless communication, in which case, the method at least comprises transmitting an acoustic signal from outside a human or animal subject, receiving the acoustic signal within the human or animal subject, and converting the acoustic signal into an optical signal.

Alternatively,however, the method may be a method of in vivo communication, in which casethe method at least comprises transmitting an acoustic signal within a human or animal subject, and converting the acoustic signal into an optical signal.

In either case, preferably, the acoustic signal is converted into an optical signal using Förster resonance energy transfer (FRET).

In embodiments, the method may further comprise converting the optical signal into an electrical signal.

Further features, goals and advantages of the present invention will now be described in association with the accompanying drawings, in which exemplary components of the invention are illustrated. Components of the devices and methods according to the invention which are at least essentially equivalent to each other with respect to their function can be marked by the same reference numerals, wherein such components do not have to be marked or described in all of the drawings.

In the following description, the invention is described by way of example only with respect to the accompanying drawings.

### Brief Description of the Drawings

Fig. 1 is a schematic diagram of an embodiment of a wireless communication system associated with a spinal column;
Fig. 2 is a schematic diagram of an embodiment of an implantable medical device; and
Fig. 3 is a schematic diagram of an embodiment of an acousto-optic transducer.

### Detailed Description of the Drawings

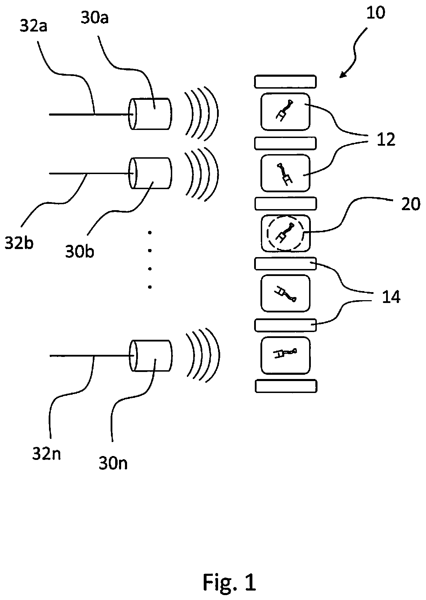

Fig. 1 schematically shows an embodiment of a wireless communication system associated with a spinal column 10. The spinal column 10 comprises a plurality of vertebrae 12 of bone, and a plurality of intervertebral discs 14 of cartilage. Adjacent ones of the vertebrae 12 are separated from each other by one of the intervertebral discs 14. The wireless communication system comprises a plurality of implantable medical devices 20 and a plurality of electro-acoustic transducers 30a, 30b, ... 30n. One or more of the implantable medical devices 20 may be implanted into any one or more of the vertebrae 12. Each of the electro-acoustic transducers 30a, 30b, ... 30n has a respective signal input line 32a, 32b, ... 32n and is able to convert an electrical signal supplied on the respective signal input line 32a, 32b, ... 32n into an acoustic signal for transdermal transmission to a respective one of the implantable medical devices 20. The electro-acoustic transducers 30a, 30b, ... 30n are configured for placement on a back of a human or animal subject, adjacent to the spinal column 10.

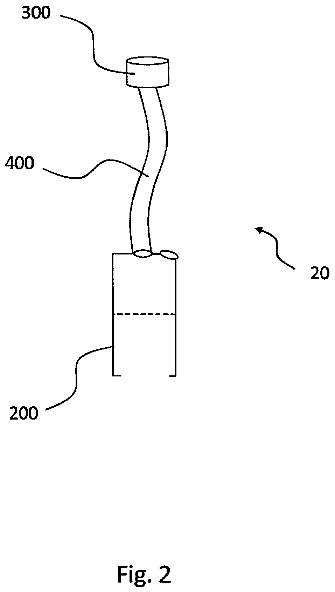

A closer view of one of the implantable medical devices 20 is schematically shown in Fig. 2. Each of the implantable medical devices 20 comprises an acousto-optic transducer 200, a photodetector 300, and an optical communication channel 400 connecting an output of the acousto-optic transducer 200 to an input of the photodetector 300. The photodetector 300 is an optoelectronic device which is able to convert a received optical signal into a transmitted electrical signal in a known fashion. The optical communication channel 400 may be a directional, low attenuation optical channel, such as a fibre optic, for example. The acousto-optic transducer 200 is a Förster resonance energy transfer (FRET) acousto-optic transducer of the type shown and described in patent application PCT/EP2017/054408, also in the name of the present applicant.

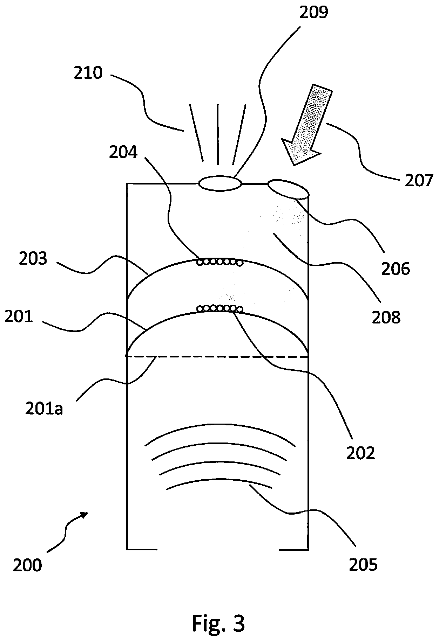

Such an acousto-optic transducer 200 is show in greater detail in Fig. 3. The acousto-optic transducer 200 comprises a graphene resonator 201, a substrate 203, an entry window 206 and an exit window 209. The graphene resonator bears a plurality of donor molecules 202 coated on its surface as a thin film. The substrate 203 bears a plurality of acceptor molecules 204 coated on the surface of the substrate 203 as a thin film facing the thin film of donor molecules 202. The donor molecules 202 and the acceptor molecules 204 may be provided as quantum dots tuned to a particular wavelength of light, for example. The graphene resonator 201 is responsive to sound waves 205 and moves during operation of the acousto-optic transducer 200 from a rest position, represented in Fig. 3 by the dashed line 201a, to a displaced position, as shown. This movement of the graphene resonator 201 brings the donor molecules 202 within range of the acceptor molecules 204 close enough for Förster resonance energy transfer (FRET) from the donor molecules 202 to the acceptor molecules 204 to take place. The entry window 206 is arranged to permit incoming ambient light 207 to fall 208 on the donor molecules 202, thereby stimulating them into an excited state. The ambient light 207 may be provided by a microscale light-emitting diode (LED) light source powered by a microcapacitor, for example. The entry window 206 can comprise a bandpass filter, in order to filter out wavelengths of ambient light 207 other than those which are required to excite the donor molecules 202. The exit window 209 is arranged to allow light 210 emitted by the acceptor molecules 204 as a result of the FRET from the donor molecules 202 to the acceptor molecules 204 to leave the acousto-optic transducer 200. The exit window 208 comprises a collimating lens to focus the emitted light 210, whence the emitted light 210 can enter the optical communication channel 400.

During operation of the wireless communication system, information transmitted on the signal input lines 32a, 32b, ... 32n is converted into corresponding acoustic signals by the electro-acoustic transducers 30a, 30b, ... 30n. These acoustic signals are transmitted transdermally to respective ones of the implantable medical devices 20, where they are converted into corresponding optical signals by one of the acousto-optic transducers 200. The optical signals travel down the optical communication channel 400 of the respective implantable medical device 20 to its photodetector 300, where the optical signals are converted into corresponding electrical signals. These electrical signals can then propagate internally through the body of a human or animal subject into which the medical devices 20 have been implanted using the body's own internal electrical conductivity and/or can be used for neural stimulation, to supply signals to one or more other implanted medical devices, such as an implanted electronic device and/or biosensor, to stimulate a prosthesis, and so on.

Whereas the exemplary embodiments shown in the accompanying drawings and described above have been given in relation to implantation in a spinal column of a subject, the implantable medical devices 20 of the wireless communication system may be implanted into other locations in a subject, as desired, including into other bones of the body than just the spinal vertebrae. The present invention is, however, particularly suited to use in association with spinal injuries. Moreover, whereas the implantable medical devices 20 have been described above as being implanted into bone, they may equally well be implanted into any other tissues of the body.

In summary, therefore, the present invention provides an implantable medical device comprising an acousto-optic transducer, a photodetector, and an optical communication channel connecting an output of the acousto-optic transducer to an input of the photodetector. The components of the implantable medical device can be made sufficiently small that they can be implanted into a living subject with ease and require no external power supply. Since the acousto-optic transducer can receive acoustic signals, which are transmissible through bone, the device can be implanted into a living bone and can be communicated with transdermally.

The invention therefore also provides a wireless communication system comprising such an implantable medical device and an electro-acoustic transducer able to transmit information to the implantable medical device by transdermal transmission of an acoustic signal. Such a system is particularly suited to the repair of spinal injuries. The invention further provides a method of transdermal wireless communication, comprising transmitting an acoustic signal from outside a human or animal subject, receiving the acoustic signal within the human or animal subject, and converting the acoustic signal into an optical signal, as well as a method of in vivo communication, comprising transmitting an acoustic signal within a human or animal subject, and converting the acoustic signal into an optical signal. Preferably, the acoustic signal is converted into an optical signal using Forster resonance energy transfer (FRET) from a donor molecule to a receptor molecule within the acousto-optic transducer.

**Reference Numerals:**

| | | | |
|---|---|---|---|
| 10 | Spinal column | 203 | Substrate |
| 12 | Vertebrae | 204 | Acceptor molecules |
| 14 | Discs of cartilage | 205 | Acoustic waves |
| 20 | Acousto-optic communication device | 206 | Entry window |
| | | 207 | Incoming ambient light |
| 30a, 30b, ... 30n | Electro-acoustictransducers | 208 | Light falling on donor molecules |
| | | 209 | Exit window |
| 200 | Acousto-optic transducer | 210 | Emitted light |
| 201 | Graphene membrane | 300 | Photodetector |
| 201a | Rest position of graphene membrane | 400 | Optical communication channel |
| 202 | Donor molecules | | |

## Claims

1. An implantable medical device (20) at least comprising:
an acousto-optic transducer (200);
a photodetector (300); and
an optical communication channel (400) connecting an output Of the acousto-optic transducer (200) to an input of the photodetector (300).

2. An implantable medical device according to claim 1, wherein the acousto-optic transducer (200) at least comprises a Forster resonance energy transfer mechanism.

3. An implantable medical device according to claim 2, wherein the acousto-optic transducer (200) at least comprises:
a graphene resonator (201) bearing at least one donor molecule (202);
a substrate (203) bearing at least one acceptor molecule (204);
the graphene resonator (201) being responsive to sound (205) to bring the at least one donor molecule within range of the at least one acceptor molecule for Forster resonance energy transfer (FRET) from the at least one donor molecule to the at least one acceptor molecule;
an entry window (206) arranged to permit incoming light (207) to fall (208) on the at least one donor molecule; and
an exit window (209) arranged to allow light (210) emitted by the at least one acceptor molecule to leave the acousto-optic transducer.

4. An implantable medical device according to claim 3, further comprising a light source to provide the incoming light (207) and a power supply for the light source.

5. An implantable medical device according to any one of claims 1 to 4, further comprising at least one of an electronic device, a biosensor and a prosthesis, each respectively having an electrical input connected to an output of the photodetector (300).

6. An implantable medical device according to any one of claims 1 to 5, further comprising at least one of an optical device and an optoelectronic device, each respectively having an optical input connected to the optical communication channel (400).

7. A wireless communication system at least comprising:
an implantable medical device (20) according to any one of claims 1 to 6; and
an electro-acoustic transducer (30a, 30b, ... 30n) able to transmit information to the implantable medical device (20) by transdermal transmission of an acoustic signal.

8. A wireless communication system according to claim 7, at least comprising:
a plurality of such implantable medical devices (20): and
a plurality of such electro-acoustic transducers (30a, 30b, ... 30n), each able to transmit information to a respective one of the implantable medical devices (20) by transdermal transmission of an acoustic signal;
wherein the plurality of implantable medical devices (20) are adapted for implantation into vertebrae of a spinal column (10); and
the plurality of electro-acoustic transducers (30a, 30b, ... 30n) are configured for placement on a back of a human or animal subject, adjacent to their spinal column (10).

9. A method of wireless communication, the method at least comprising:
transmitting an acoustic signal;
receiving the acoustic signal within a human or animal subject; and
converting the acoustic signal into an optical signal.

10. A method of wireless communication according to claim 9, wherein the method is a method of transdermal wireless communication and transmitting an acoustic signal at least comprises transmitting the acoustic signal from outside the human or animal subject.

11. A method of wireless communication according to claim 9, wherein the method is a method of in vivo communication and transmitting an acoustic signal at least comprises transmitting the acoustic signal within the human or animal subject.

12. A method of wireless communication according to any one of claims 9 to 11, wherein the acoustic signal is converted into an optical signal using Forster resonance energy transfer (FRET).

13. A method of wireless communication according to any one of claims 9 to 12, further comprising converting the optical signal into an electrical signal.

## Patentansprüche

1. Eine implantierbare medizinische Vorrichtung (20), die mindestens aufweist:
einen akusto-optischen Wandler (200)
einen Photodetektor (300); und
einen optischen Kommunikationskanal (400), der einen Ausgang des akusto-optischen Wandlers (200) mit einem Eingang des Fotodetektors (300) verbindet.

2. Implantierbare medizinische Vorrichtung gemäß Anspruch 1, wobei der akustooptische Wandler (200) zumindest einen Förster-Resonanz-Energieübertragungsmechanismus aufweist.

3. Implantierbare medizinische Vorrichtung gemäß Anspruch 2, wobei der akustooptische Wandler (200) mindestens aufweist:
einen Graphen-Resonator (201), der mindestens ein Donor-Molekül (202) trägt
ein Substrat (203), das mindestens ein Akzeptormolekül (204) trägt;
wobei der Graphen-Resonator (201) auf Schall (205) anspricht, um das mindestens eine Donor-Molekül in Reichweite des mindestens einen Akzeptor-Moleküls für den Förster-Resonanz-Energie-Transfer (FRET) von dem mindestens einen Donor-Molekül zu dem mindestens einen Akzeptor-Molekül zu bringen;
ein Eintrittsfenster (206), das so angeordnet ist, dass einfallendes Licht (207) auf das mindestens eine Donormolekül fallen kann (208); und
ein Austrittsfenster (209), das so angeordnet ist, dass es Licht (210), das von dem mindestens einen Akzeptormolekül emittiert wird, erlaubt, den akusto-optischen Wandler zu verlassen.

4. Implantierbare medizinische Vorrichtung gemäß Anspruch 3, die ferner eine Lichtquelle zum Bereitstellen des einfallenden Lichts (207) und eine Energieversorgung für die Lichtquelle aufweist.

5. Implantierbare medizinische Vorrichtung gemäß einem der Ansprüche 1 bis 4, die ferner mindestens eine elektronische Vorrichtung, einen Biosensor und eine Prothese aufweist, die jeweils einen elektrischen Eingang haben, der mit einem Ausgang des Photodetektors (300) verbunden ist.

6. Implantierbare medizinische Vorrichtung gemäß einem der Ansprüche 1 bis 5, die ferner mindestens eine optische Vorrichtung und eine optoelektronische Vorrichtung aufweist, die jeweils einen optischen Eingang haben, der mit dem optischen Kommunikationskanal (400) verbunden ist.

7. Ein drahtloses Kommunikationssystem, das mindestens aufweist:
eine implantierbare medizinische Vorrichtung (20) gemäß einem der Ansprüche 1 bis 6; und
einen elektroakustischen Wandler (30a, 30b, ... 30n), der in der Lage ist, durch transdermale Übertragung eines akustischen Signals Informationen an die implantierbare medizinische Vorrichtung (20) zu übertragen.

8. Ein drahtloses Kommunikationssystem gemäß Anspruch 7, das mindestens aufweist:
eine Mehrzahl solcher implantierbarer medizinischer Vorrichtungen (20); und
eine Mehrzahl solcher elektroakustischer Wandler (30a, 30b, ... 30n), die jeweils in der Lage sind, Informationen an eine jeweilige der implantierbaren medizinischen Vorrichtungen (20) durch transdermale Übertragung eines akustischen Signals zu übertragen;
wobei die mehreren implantierbaren medizinischen Vorrichtungen (20) zur Implantation in Wirbel einer Wirbelsäule (10) geeignet sind; und
die Mehrzahl der elektroakustischen Wandler (30a, 30b, ... 30n) zur Platzierung auf einem Rücken eines menschlichen oder tierischen Subjekts, angrenzend an dessen Wirbelsäule (10), konfiguriert sind.

9. Ein Verfahren zur drahtlosen Kommunikation, wobei das Verfahren mindestens aufweist:
Aussenden eines akustischen Signals;
Empfangen des akustischen Signals innerhalb eines menschlichen oder tierischen Subjekts; und
Umwandlung des akustischen Signals in ein optisches Signal.

10. Verfahren zur drahtlosen Kommunikation gemäß Anspruch 9, wobei das Verfahren ein Verfahren zur transdermalen drahtlosen Kommunikation ist und das Übertragen eines akustischen Signals zumindest das Übertragen des akustischen Signals von außerhalb des menschlichen oder tierischen Subjekts aufweist.

11. Verfahren zur drahtlosen Kommunikation gemäß Anspruch 9, wobei das Verfahren ein Verfahren zur In-vivo-Kommunikation ist und das Übertragen eines akustischen Signals zumindest das Übertragen des akustischen Signals innerhalb des menschlichen oder tierischen Subjekts aufweist.

12. Verfahren zur drahtlosen Kommunikation gemäß einem der Ansprüche 9 bis 11, wobei das akustische Signal unter Verwendung von Förster-Resonanz-Energie-Transfer (FRET) in ein optisches Signal umgewandelt wird.

13. Verfahren zur drahtlosen Kommunikation gemäß einem der Ansprüche 9 bis 12, das ferner die Umwandlung des optischen Signals in ein elektrisches Signal aufweist.

## Revendications

1. Un dispositif médical implantable (20) comprenant au moins :
un transducteur acousto-optique (200) ;
un photodétecteur (300) ; et
un canal de communication optique (400) reliant une sortie du transducteur acousto-optique (200) à une entrée du photodétecteur (300).

2. Dispositif médical implantable selon la revendication 1, dans lequel le transducteur acousto-optique (200) comprend au moins un mécanisme de transfert d'énergie de résonance de Förster.

3. Dispositif médical implantable selon la revendication 2, dans lequel le transducteur acousto-optique (200) comprend au moins un mécanisme de transfert d'énergie de résonance de Förster. 3 :
un résonateur au graphène (201) portant au moins une molécule donneuse (202);
un substrat (203) portant au moins une molécule acceptrice (204) ;
le résonateur au graphène (201) étant sensible au son (205) pour amener la au moins une molécule donneuse dans la gamme de la au moins une molécule acceptrice pour le transfert d'énergie de résonance de Förster (FRET) de la au moins une molécule donneuse à la au moins une molécule acceptrice ;
une fenêtre d'entrée (206) disposée de manière à permettre à la lumière entrante (207) de tomber (208) sur la molécule donneuse au nombre d'au moins une ; et
une fenêtre de sortie (209) disposée de manière à permettre à la lumière (210) émise par la ou les molécules acceptrices de quitter le transducteur acousto-optique.

4. Dispositif médical implantable selon la revendication 3, comprenant en outre une source de lumière pour fournir la lumière entrante (207) et une alimentation électrique pour la source de lumière.

5. Dispositif médical implantable selon l'une quelconque des revendications 1 à 4, comprenant en outre au moins un dispositif électronique, un biocapteur et une prothèse, chacun ayant respectivement une entrée électrique connectée à une sortie du photodétecteur (300).

6. Dispositif médical implantable selon l'une quelconque des revendications 1 à 5, comprenant en outre au moins l'un d'un dispositif optique et d'un dispositif optoélectronique, chacun ayant respectivement une entrée optique connectée au canal de communication optique (400).

7. Un système de communication sans fil comprenant au moins :
un dispositif médical implantable (20) selon l'une quelconque des revendications 1 à 6 ; et
un transducteur électro-acoustique (30a, 30b, ... 30n) capable de transmettre des informations au dispositif médical implantable (20) par transmission transdermique d'un signal acoustique.

8. Système de communication sans fil selon la revendication 7, comprenant au moins :
une pluralité de tels dispositifs médicaux implantables (20) ; et
une pluralité de ces transducteurs électro-acoustiques (30a, 30b, ... 30n), chacun étant capable de transmettre des informations à l'un des dispositifs médicaux implantables (20) par transmission transdermique d'un signal acoustique ;
dans lequel la pluralité de dispositifs médicaux implantables (20) sont adaptés pour être implantés dans des vertèbres d'une colonne vertébrale (10) ; et
la pluralité de transducteurs électro-acoustiques (30a, 30b, ... 30n) sont configurés pour être placés sur le dos d'un sujet humain ou animal, à proximité de sa colonne vertébrale (10).

9. Une méthode de communication sans fil, la méthode comprenant au moins :
la transmission d'un signal acoustique ;
la réception du signal acoustique chez un sujet humain ou animal ; et
la conversion du signal acoustique en un signal optique.

10. Méthode de communication sans fil selon la revendication 9, dans laquelle la méthode est une méthode de communication sans fil transdermique et la transmission d'un signal acoustique comprend au moins la transmission du signal acoustique depuis l'extérieur du sujet humain ou animal.

11. Méthode de communication sans fil selon la revendication 9, dans laquelle la méthode est une méthode de communication in vivo et la transmission d'un signal acoustique comprend au moins la transmission du signal acoustique à l'intérieur du sujet humain ou animal.

12. Méthode de communication sans fil selon l'une quelconque des revendications 9 à 11, dans laquelle le signal acoustique est converti en un signal optique en utilisant le transfert d'énergie de résonance de Förster (FRET).

13. Méthode de communication sans fil selon l'une quelconque des revendications 9 à 12, comprenant en outre la conversion du signal optique en un signal électrique.
